# EUROPEAN PATENT SPECIFICATION

(11) **EP 2 221 005 B1**
(45) Date of publication and mention of the grant of the patent: **19.11.2014**
(21) Application number: 09153135.0
(22) Date of filing: 18.02.2009
(51) Int. Cl.: A61B 17/02, A61B 1/00

(54) **Endoscopic retractor**
Endoskopischer Retraktor
Écarteur endoscopique

(43) Date of publication of application: 25.08.2010
(73) Proprietor: University Of Dundee, Dundee, Scotland Angus DD1 4HN (GB)
(72) Inventor: Frank, Timothy Graham, Dr., Newport-On-Tay Fife DD68NG, Scotland (GB); Gove, James, Dundee, DD4 9AJ, Scotland (GB); Cuschieri, Sir Alfred, St. Andrews, Fife KY16 9TY, Scotland (GB)
(74) Representative: Witte, Weller & Partner Patentanwälte mbB

(56) References cited:
- EP-A- 0 316 816
- WO-A-93/22973
- WO-A-96/39915
- WO-A-98/14124
- DE-C1- 4 318 993
- DE-U1- 9 102 759
- US-A- 5 258 026
- US-A- 5 782 859
- US-A1- 2004 082 969

## Description

The present invention relates to an endoscopic retractor comprising a shaft having a distal end, a proximal end and a central axis, at least two retractor fingers arranged at the distal end of the shaft whereby the at least two retractor fingers are movable between a closed position in which the at least two movable retractor fingers are substantially parallel to the central axis of the shaft and an open position in which the at least two movable retractor fingers are of an angle to the central axis of the shaft and whereby in the closed position the at least two movable retractor fingers are substantially parallel to each other and in the open position the at least two movable retractor fingers are at an angle to each other in a plane which is at angle to the central axis of the shaft, a handle arranged at the proximal end of the shaft, an actuating element designed as a tube concentric to the shaft connecting the at least one movable retractor finger with the handle, so that the at least one movable retractor finger can be moved between the open position and the closed position by means of the handle and at least one channel for inserting an endoscopic instrument running along the central axis of the shaft and connecting an opening in the vicinity of the proximal end of the shaft with an opening in the vicinity of the distal end of the shaft.

The invention further relates to an endoscope.

Endoscopic retractors as described above are known, for example, from EP 0 614 646 B1 where they are referred to as surgical trocar sleeves.

WO 98/14124, which is considered to be the closest prior art, discloses a surgical instrument for endoscopic and general surgery having manual controls and a single handed design. The endoscopical surgical instrument described may also be configured to include electrocautery capability, an integrated endoscope and irrigation and aspiration capabilities.

DE 43 18 993 C1 discloses an endoscopic instrument for the manual examination of tissue comprising a shaft with probing fingers at the distal end and a handle at the proximal end.

WO 96/39915 discloses a surgical instrument for use with a viewing system. The surgical instrument described can readily be assembled and includes an elongated shaft and a flexible endoscope or optical assembly that can be readily and removably secured along the length of the entire length of the shaft.

US 2004/0082969 A1 discloses a laparoscopic direct vision dissecting port for providing safe entry into a body cavity as well as being operative to serve as a standard laparoscopic port for using laparoscopic surgery. The device comprises an elongate laparoscopic port within which a laparoscope may be positioned. The distal end of the tip is transparent in nature and operatively transitional between a closed configuration and an open operative configuration the latter of which causing tissue to be selectively dissected in layer-by-layer fashion while under direct vision of the laparoscope disposed therein.

DE 91 02 759 U1 discloses an endoscopic instrument comprising a singular retractor finger whose operation is simply driven by gravity.

WO 93/22973 discloses a retractor for use during endoscopic surgery. Bladed instruments located inside the patient's body at an insertion end of the retractor are manipulated by controls located outside the body at a control end. The blades may be moved in various combinations being extended away from the longitudinal axis of the retractor body and/or spread apart. The blades according to this document may be actuated with controls which extend from the control end of the body.

In minimally invasive laparoscopic surgery, surgical procedures are carried out within the body of the patient without the need to fully cut open the abdomen of a patient. In order to insert the necessary surgical instruments, small incisions are made into the abdomen, for example using trocars. In order to create space to carry out a surgical procedure within the abdominal cavity, the abdominal cavity is hereby usually insufflated using an inert gas, for example CO₂. During some procedures, a number of surgical instruments must be introduced into the body of a patient leading to the need to create an equal number of incisions in the abdomen. Such incisions lead to an increased stress on the patient and can cause scarring later on.

In order to reduce the number of surgical instruments that must be introduced into the body of a patient and therefore the number of incisions that have to be made into the abdomen, attempts have been made to create endoscopic instruments having multiple functionalities.

The above-mentioned EP 0 614 646 B1 describes what is referred to as a surgical trocar sleeve, i.e. an instrument with a shaft comprising a channel connecting an opening in the vicinity of the proximal end of the shaft with an opening in the vicinity of the distal end of the shaft, that has been provided with retractor fingers that can be moved between a closed position and an open position by means of an actuating element. In the endoscopic instrument described in this document the retractor fingers are arranged around the opening at the distal end of the shaft, which in this case is formed by the open end of the tubular shaft.

In practice, it has been shown that mounting the retractor fingers in the way that is described in EP 0 614 646 B1 is problematic. If the retractor fingers are connected to the shaft by hinges mounted on the sidewall of a shaft of an endoscopic instrument having the usual wall thickness, the resulting connection between the shaft and the retractor fingers is quite weak and the amount of force that can be transmitted onto the tissue via the retractor fingers is extremely limited. This is particularly problematic in those situations where organs, such as for example, the liver, which can weigh around 1.5 kgs need to be moved with the retractor fingers.

If the connections between the retractor fingers and the shaft according to EP 0 614 646 B1 are to be strengthened, a shaft would have to be used that has a distinctly thicker wall which would either decrease the internal diameter for a given outer diameter with the associated risks of no longer being able to insert a standard endoscopic instrument through the shaft or increase the outer diameter for a given inner diameter, which would a) necessitate bigger incisions and therefore increase the stress on a patient and b) would run the risk of no longer being able to introduce the instrument through a standard trocar. Not being able to insert such an endoscopic instrument through a standard trocar anymore would necessitate either the production of non-standard trocars with the associated costs or the use of such an instrument without a trocar. Since the instrument is also used as a retractor a certain amount of forward and backward movement is necessary in order to move the desired tissue, which can lead to friction with the abdominal wall and further stress for the patient and therefore the use of the instrument without a trocar is also not desirable.

It is therefore one aim of the present invention to provide endoscopic instruments with which several functions can be performed in the body of the patient, in which retractor fingers are attached to the shaft with sufficient strength to move even heavy internal organs without affecting the internal and external diameter of the endoscopic instrument.

According to the invention the problem is solved by providing an endoscopic retractor as described earlier in which the opening in the vicinity of the distal end of the shaft is realized as a cutaway in a sidewall of the shaft and in which the at least two retractor fingers are mounted distally of the opening in the vicinity of the distal end of the shaft at the distal end of the shaft.

Furthermore, the problem is solved by an endoscope comprising a shaft having a distal end, a proximal end and a central axis, image transfer means for transferring images from the distal end of the shaft to the proximal end of the shaft, a handle arranged at the proximal end of the shaft, at least two retractor fingers arranged at the distal end of the shaft, whereby the at least two retractor fingers are movable between a closed position in which the at least two movable retractor fingers are substantially parallel to the central axis of the shaft and an open position in which the at least two movable retractor fingers are at an angle to the central axis of the shaft and whereby in the closed position the at last two movable retractor fingers are substantially parallel to each other and in the open position the at least two movable retractor fingers are at an angle to each other in a plane which is at an angle to the central axis at the shaft and an actuating element designed as a tube concentric to the shaft connecting the at least one movable retractor finger with the handle so that the at least one movable retractor finger can be moved between the open position and the closed position by means of the handle whereby the distal end of the image transfer means ends in an opening in the shaft in the vicinity of the distal end of the shaft through which image information is acquired and which is realized as a cutaway in a sidewall of the shaft and whereby the at least two retractor fingers are mounted distally of the opening in the vicinity of the distal end of the shaft at the distal end of the shaft.

The present inventors have surprisingly found that by providing the endoscopic instruments of the invention with an opening in the shape of a cutaway in a sidewall of the shaft, useful access to the abdomen of the patient can be created. This, in particular, if the endoscopic retractor is used in combination with an endoscope with 30° or 60° sideways optics. Even in combination with an endoscope with straight ahead optics it has been shown that very little optical information is lost. Since the retractor fingers are mounted at the distal end of the shaft, and since this distal end is no longer used as the distal opening of the channel running through the shaft or the viewing system, a mounting block can be arranged at distal end which forms a secure and stable mounting system for the retractor fingers which can take substantially higher loads than the mounting system known from the state of the art.

For the purpose of the invention the expression "endoscopic instrument" refers to all instruments which are usually inserted into the body of the patient during minimally invasive surgery for diagnostic or therapeutic purposes. Examples for such instruments include endoscopic forceps, endoscopic retractors, endoscopic scissors, HF-loops or endoscopes.

For the purpose of the invention, the expression "image transfer means" refers to any system which transfers images in whatever form from the distal end of the endoscopic instrument to the proximal end of the endoscopic instrument. Such image transfer systems include for example conventional optics, optics including rod lenses, bundles of optical fibers or systems in which image capture electronics such as CCD chips at the distal end of the endoscope directly convert optical information into electrical impulses, and transfer those through wires to the proximal end of the endoscope, where the electrical impulses are again converted into optical information.

The expression "lighting means" refers to any system which is capable of providing light at the distal end of the endoscopic instrument. Such systems are usually, but not exclusively, based on bundles of optical fibers, which are connected via one or several connectors to an endoscopic light source. Also included, however are systems comprising for example LED-based light sources arranged at the distal end of the endoscopic instrument, which are connected to a power source at the proximal end of the endoscopic instrument by electrical wires.

In a further embodiment, the endoscopic retractor or the endoscope comprises at least one fixed retractor finger extending from the distal end of the shaft substantially parallel to the central axis of the shaft.

By having a retractor finger fixedly attached to the distal end of the shaft and extending parallel to the central axis of the shaft, an instrument is created which has about the same cross-section as an instrument not having a retractor finger and which at the same time is provided with means for moving tissue from an area, which for example needs to be viewed with an endoscope. Furthermore, fixedly attaching a retractor finger to the distal end of the endoscopic retractor or endoscope has got the advantage on the one hand that such a configuration is easy to manufacture and on the other hand since no movable connections are needed, a very firm and stable connection can be achieved between the retractor finger and the shaft.

A combination of at least two movable and one fixed retractor finger and in particular of two movable and one fixed retractor finger has proved to be particularly well suited for the endoscopic retractors and endoscopes of the invention.

In a further embodiment, the endoscopic retractor comprises sealing means arranged around the inner circumference of the channel.

Such sealing means can, for example, take the shape of an O-ring arranged on the inside of the channel. Such an O-ring is thereby preferably made from an elastic polymer such as for example silicone.

The provision of sealing means around the inner circumference of the channel has got the advantage that they aid in avoiding gas leaks, for example from the abdomen of a patient, while the endoscopic retractor of the invention is inserted into the body of a patient.

In a further embodiment of the invention, the endoscopic retractor comprises locking means for locking an endoscopic instrument that has been inserted into the channel into place and in particular the endoscopic instrument can be locked into place in any angular orientation with regards to the shaft.

Such a design has the advantage that the endoscopic retractor of the invention and the endoscopic instrument inserted therein can be combined into one firmly connected unit making the handling of the unit easier for the operator. If the endoscopic instrument can be locked into place in any angular orientation with regards to the shaft, also none of the flexibility of two separate units which are not locked together is lost.

In a further embodiment, the endoscopic retractor comprises image transfer means for transferring images from the distal end of the shaft to the proximal end of the shaft.

By providing an endoscopic retractor of the invention with image transfer means the endoscopic retractor is also given the function of an endoscope, thereby combining two functions into one instrument with the possibility of adding a third function by inserting another endoscopic instrument through the channel in the endoscopic retractor making such an instrument even more versatile.

In a further embodiment, the endoscopic retractor comprises lighting means for providing light at the distal end of the shaft.

By providing an endoscopic retractor of the invention with lighting means such an instrument can be used to light up an area viewed with an endoscope.

The endoscopic retractor comprises a handle arranged at the proximal end of the shaft and an actuating element connecting the at least one movable retractor finger with the handle so that the at least one movable retractor finger can be moved between the open position and the closed position by means of the handle.

The actuating element is designed as a tube concentric to the shaft.

The use of a tube concentric to the shaft has got the advantage that the force exerted onto the retractor fingers can be distributed over the whole circumference of the tube making this a particularly stable and reliable way of transferring the force from the handle to the retractor fingers.

Furthermore, by having the actuating element designed as a tube concentric to the shaft of the endoscopic retractor or the endoscope, an instrument can be created that is particularly easy to dismantle, for example in order to clean it.

This construction further has the advantage that such an instrument can be kept gas-tight easily, thereby avoiding the leakage of insufflation gas.

In a further embodiment of the invention, the handle is designed as a lever.

Experience has shown that the use of a lever as a handle is a particularly intuitive design, since it can be designed in such a way that the tilting of the lever directly corresponds to the movement of the retractor fingers, further increasing the safety of the use of an endoscopic retractor or endoscope of the invention.

In a further embodiment the endoscope comprises lighting means for providing light at the distal end of the shaft.

By providing an endoscope of the invention with lighting means the area to be viewed with the endoscope can be lighted directly and with one instrument without the need to insert further instruments and the associated need to provide further incisions in the abdomen of a patient.

It is understood that the above-mentioned features and features to be explained below can be used not only in the combinations specified but also in other combinations or on their own without departing from the scope of the appended claims.

The invention is now described in more detail and explained below on the basis of selected exemplary retractors in conjunction with the accompanying drawings in which:
- Fig. 1: shows a perspective view of an endoscopic retractor with the retractor fingers in the closed position;
- Fig. 2: shows a perspective view of the endoscopic retractor of Fig. 1 with the retractor fingers in the open position;
- Fig. 3: shows a side view of the endoscopic retractor of Fig. 1 with the retrac- tor fingers in the open position in section;
- Fig. 4: shows a perspective view of an embodiment of an endoscope with the retractor fingers in the closed position;
- Fig. 5: shows a perspective view of the endoscope of Fig. 4 with the retractor fingers in the open position;
- Fig. 6: shows a side view of an exemplary endoscopic retractor with the retractor fingers in the closed position in section; and
- Fig. 7: shows a side view of the endoscopic retractor of Fig. 6 with the retrac- tor fingers in the open position in section.

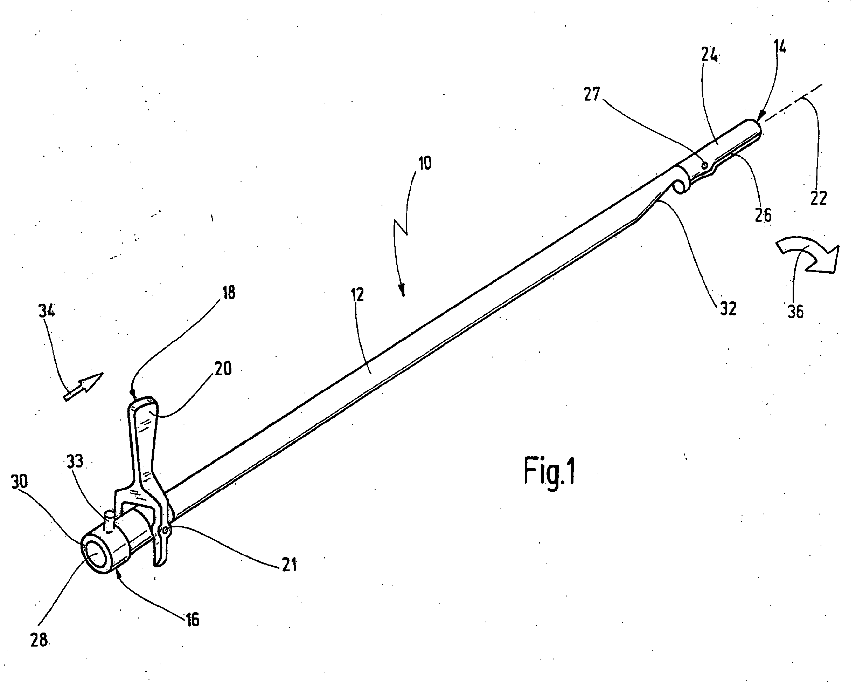

In Fig. 1 an endoscopic retractor in its entirety is denoted with the reference numeral 10.

The endoscopic retractor 10 comprises a shaft 12 having a distal end 14 and a proximal end 16. Arranged at the proximal end 16 is a handle 18 which in this case takes the form of a lever 20. The lever 20 is connected to the shaft 12 of the endoscopic retractor 10 by a pivot axis 21.

Arranged at the distal end 14 of the shaft 12 are two retractor fingers 24 and 26, whereby retractor finger 24 is fixedly connected to shaft 12 and extends substantially parallel to a central axis 22 of the shaft 12 which is represented here by a broken line.

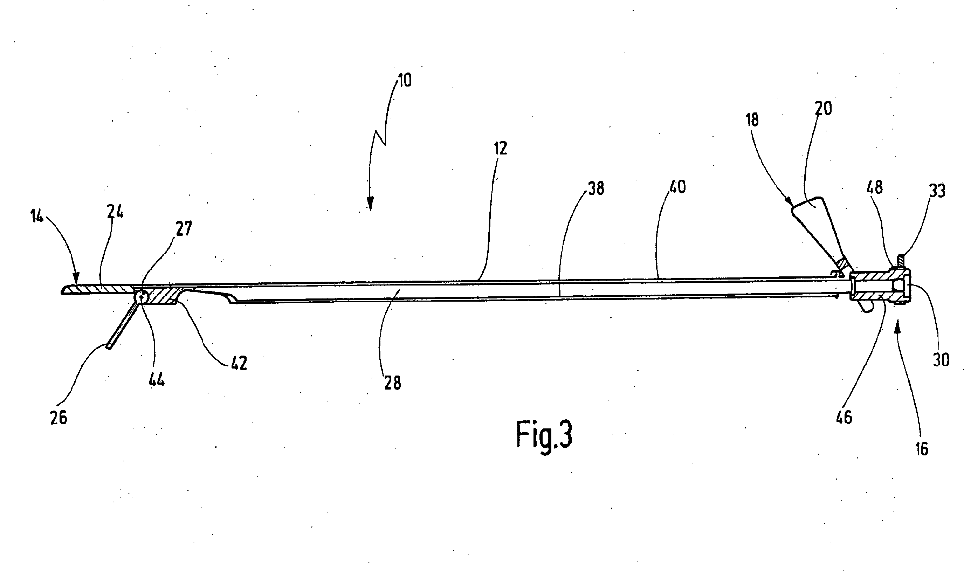

Retractor finger 26 is movably connected to the shaft 12 by a pivot axis not shown here. The movable retractor finger 26 is further connected to the handle 18 via an actuating element in form of a tube which is concentric to the shaft 12 and forms the outer tube of the shaft 12. The retractor finger 26 is connected to a distal section of the actuating element by a connecting stud 27. The function of the assembly of retractor finger 26, actuating element and handle 18 will be described in more detail in connection with Fig. 3.

On the inside of the shaft 12 runs a channel 28 which connects an opening 30 at the proximal end 16 of the endoscopic retractor 10 with an opening 32 in the vicinity of the distal end 14 of the shaft 12. The opening 32 is hereby realized as a cutaway.

While in use, the endoscopic retractor 10 can either first be introduced into the body of a patient on its own or can be combined with an endoscopic instrument and can then be inserted into the body of a patient through an insertion port arranged there earlier. A small stud 33 is thereby provided at the proximal end 16 of the shaft 12, which can be used to fixedly attach the endoscopic retractor 10 to any endoscopic instrument inserted into channel 28 of shaft 12. The stud 33 is hereby rotatably connected to the shaft 12 so that the connection can be made in any angular orientation with regards to the shaft 12 and that the orientation can be changed during the operation itself. Preferably such an endoscopic instrument is an endoscope and due to the nature of the opening 32 in the vicinity of the distal end 14 of the shaft 12 it is in particular an endoscope which is provided with sideways optics, for example having a 30 degree or 60 degree viewing angle.

Once the endoscopic retractor 10 has been inserted into the body of the patient, the lever 20 is pushed forwards as indicated here by arrow 34. This pushing forward of the lever 20 will push the actuating element forwards and exert a pushing force onto the movable retractor finger 26. Since the retractor finger 26 is fixedly connected to the distal area of shaft 12 by a pivot axis, the forward pushing motion is converted into a pivoting motion as indicated by arrow 36.

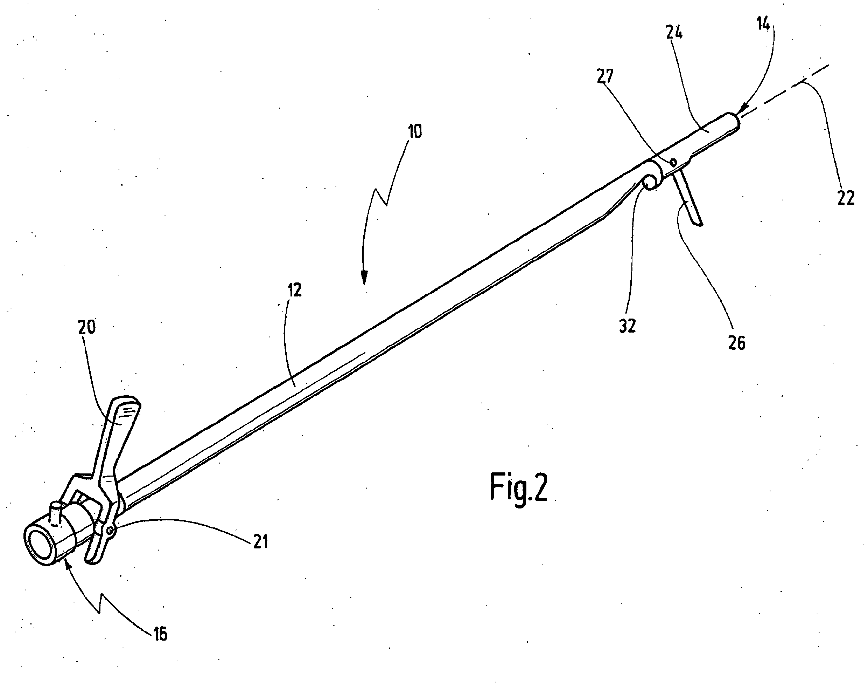

The situation after the lever 20 has been pushed forwards can be seen in Fig. 2, where it can be seen that the movable retractor finger 26 has pivoted around its pivot axis and is now at an angle to the central axis 22 of the shaft 12. This can be done when the endoscopic retractor is in place, for example if one wishes to pull tissue to be investigated and/or treated closer to the opening 32 or it can be performed prior to reaching the final position of the endoscopic retractor in order to create a larger surface area with which one can push tissue away from the site to be observed or treated.

The function of the assembly of retractor finger 26, actuating element and handle 18 becomes particularly easy to understand with reference to Fig. 3. Fig. 3 shows the endoscopic retractor 10 of Figure 1 in section. It hereby becomes clear that the shaft 12 consists of two tubes, an inner tube 38 and an outer tube 40 which in this case forms the actuating element. The inner tube 38 hereby forms the channel 28 through which an endoscopic instrument can be inserted.

In the vicinity of the distal end 14 of the endoscopic retractor 10, a mounting block 42 is attached to the inner tube 38 on which the movable retractor finger 26 is mounted on a pivot axis which is indicated here with reference numeral 44. The movable retractor finger 26 is further connected to the outer tube 40 through the connecting stud 27. If the outer tube 40 and the inner tube 38 are moved relative to one another in an axial direction, this movement is translated into a pivoting movement of the movable retractor finger 26, and with this movement the movable retractor finger 26 can be moved between the closed position and open position shown here.

In the vicinity of the proximal end 16 of the endoscopic retractor 10, the inner tube 38 is connected to an end piece 46 which also comprises the proximal opening 30 of the endoscopic retractor 10. The connection between the inner tube 38 and the end piece 46 in the present case is achieved by soldering, but it can also be achieved by gluing, welding, screwing or other techniques known to a man of the art.

Further connected to the end piece 46 is the handle 18 in form of the lever 20 by a pivot axis not shown in this figure. The lever 20 is further connected to the proximal end of the outer tube 40. By tilting the lever 20, the outer tube 40 can be moved relative to the inner tube 38 in an axial direction. As already described earlier, such an axial movement of the outer tube 40 relative to the inner tube 38 is translated at the distal end of the endoscopic retractor 10 into a pivoting movement of the movable retractor finger 26 and this way the movable retractor finger 26 can be moved between an open position and a closed position by moving the handle 18.

Furthermore, it can be seen from this representation that the small stud 33 is connected to the end piece 46 of the endoscopic retractor 10 via a ring 48, and through this connection the small stud 33 and by extension any endoscopic instrument connected thereto can be rotated to any angular orientation with regards to the endoscopic retractor 10.

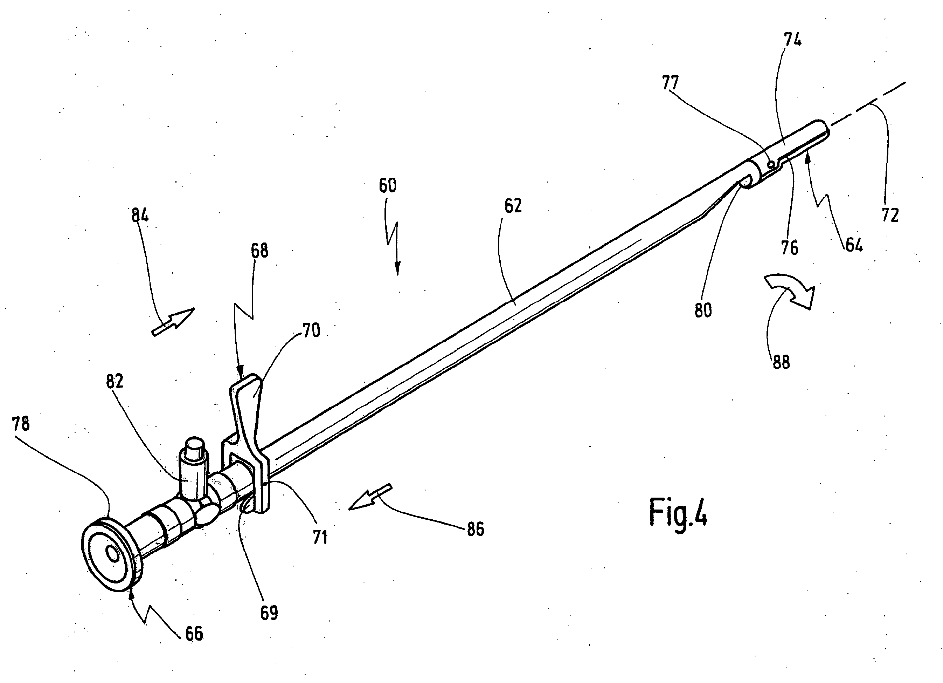

In Fig. 4 an embodiment of an endoscope is denoted in its entirety with the reference numeral 60.

The endoscope 60 comprises a shaft 62 having a distal end 64 and a proximal end 66. Arranged at the proximal end 66 is a handle 68 which is connected to an actuating element 69 of which only the very proximal end is visible here. This actuating element here takes the form of a tubular control rod which runs inside the shaft 62.

The handle 68 here takes the form of a lever 70 which is connected to the shaft 62 by a pivot axis 71.

Arranged at the distal end 64 of the shaft 62 is a retractor finger 74 which is fixedly attached to shaft 62 and which runs substantially parallel to a central axis 72 of the shaft 62 which is represented here by a broken line.

Furthermore, attached to the distal end 64 of the shaft 62 are two movable retractor fingers of which in this figure only one retractor finger 76 is visible. This retractor finger 76 is connected to the distal end 64 of the shaft 62 via a pivot axis 77. The second retractor finger which is not visible in this figure is also connected to the distal end 64 of the shaft 62 by a pivot axis, whereby the two pivot axes are inclined with respect to each other.

The endoscope 60 further comprises image transmitting means which in this case comprise an eye piece 78 which is connected via a rod lens-type optical system to a prism which is arranged in a cutaway 80 in the vicinity of a distal end 64 of the shaft 62. Through the combination of the prism and the rod lenses image information from the distal end 64 of the shaft 62 is transmitted to the proximal end 66 of the shaft and can there be viewed by an operator by looking into the eye piece 78.

The endoscope 60 further comprises lighting means which hereby comprise a connector 82 for connecting the endoscope 64 to an endoscopic light source and a set of fiber optic bundles which run inside the shaft 62 of the endoscope 60 from the connector 82 to the cutaway 80 in the vicinity of the distal end 64 of the shaft 62. With the aid of such a system, the field of vision of the image transfer means can be illuminated.

In use the endoscope 60 is introduced into the body of a patient in the closed position of the retractor fingers 76. If an operator wants to move the movable retractor fingers 76 from the closed position to the open position, he can push the lever 70 forwards in the direction of arrow 84, whereby the lever 70 pivots around the pivot axis 71 and pulls the actuating element 69 backwards in the direction of arrow 86.

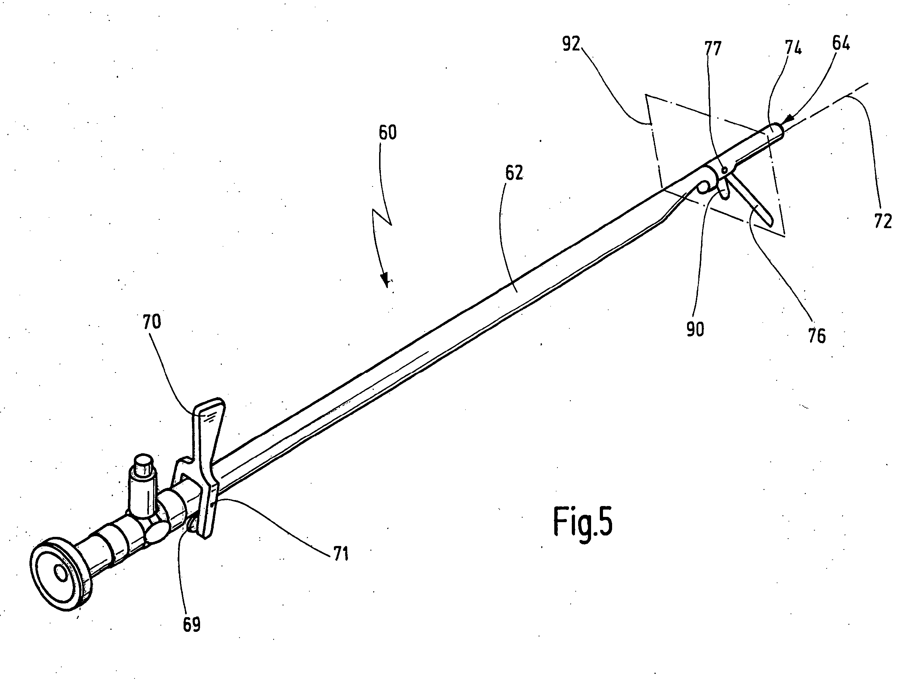

The actuating element 69 is connected to the movable retractor fingers 76 at the point distal from the pivot axis 77. This arrangement will ensure that if a pulling force is exerted onto the actuating element 69, this is converted into a pivoting motion in the direction of arrow 88 and will move the retractor fingers 76 from the closed position to the open position as shown in Fig. 5.

In the open position as shown in Fig. 5, the second movable retractor finger 90 now becomes visible.

In the open position depicted here, both retractor fingers 76, 90 are at an angle to the central axis 72 of the shaft 62.

Due to the fact that the pivot axis 77 of the movable retractor finger 76 is inclined to the pivot axis of the movable retractor finger 90, the two movable retractor fingers 76 and 90 do not only pivot away from the central axis 72 of the shaft 62 during the pivoting motion indicated by arrow 68 in Fig. 4, but also away from each other, so that they are now at an angle to each other in a plane 92, which is indicated here by dash-dotted lines and which itself is at an angle to the central axis 72 of the shaft 62. This moves the two movable retractor fingers 76 and 90 from a substantially parallel configuration to a V-shaped configuration. This increases the size of the surface over which forces are exerted on the tissue and minimizes any damage done to the tissue.

The movement from the closed position to the open position can either take place once the endoscope 60 is in place, for example to pull tissue closer to the endoscope 60 for inspection or before the endoscope 60 has reached its final position, in order to push tissue away from a site to be inspected by the endoscope 60.

Once the observations are over, the operator can start withdrawing the endoscope 60, while at the same time pulling lever 70 back in the direction opposite to the direction indicated by arrow 84 in Fig. 4 which will return the movable retractor fingers 76 and 90 back to the closed position, so that the endoscope 60 can be withdrawn from the body of a patient without causing any damage in the process.

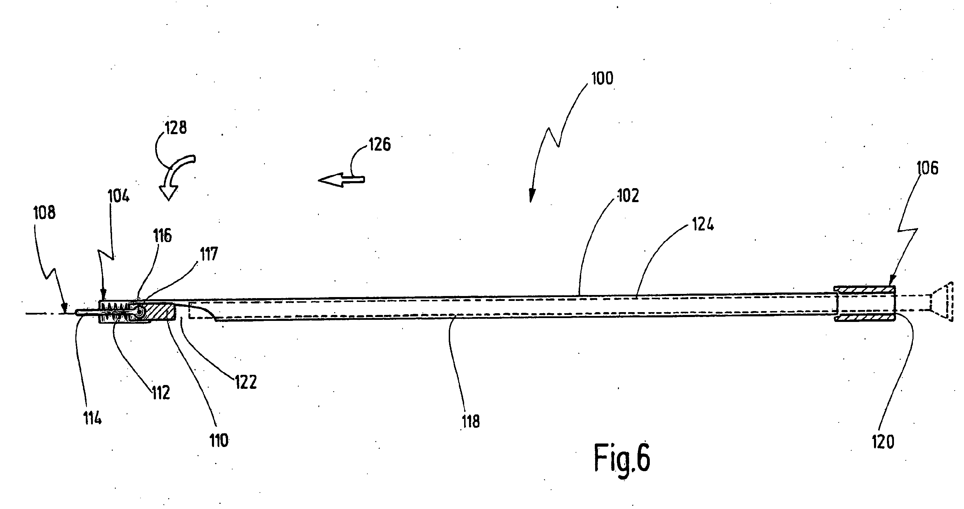

In Fig. 6 an exemplary endoscopic retractor in its entirety is denoted with the reference numeral 100.

The endoscopic retractor 100 comprises a shaft 102 having a distal end 104, a proximal end 106 and a central axis 108 shown here as a dash-dotted line

Arranged within the distal end 104 of the shaft 102 is a mounting block 110 which abuts against a helical spring 112 which in turn abuts against the closed distal end 104 of the shaft 102.

Furthermore, attached to the distal end 104 of the shaft 102 are two movable retractor fingers of which in this Figure only one retractor finger 114 is visible. This retractor finger 114 is connected to the distal end of the shaft 102 via a pivot axis 116. The second retractor finger which is not visible in this Figure is also connected to the distal end 104 of the shaft 102 by a pivot axis whereby the two pivot axis are inclined with respect to each other.

The inside of the shaft 102 furthermore forms a channel 118 through which an endoscopic instrument can be inserted. The channel 118 connects an opening 120 in the vicinity of the proximal end 106 of the shaft 102 with an opening 122 in the vicinity of the distal end 104 of the shaft 102.

As can be seen here the opening 122 is realized as a cutaway and is arranged proximally of the retractor fingers 114.

In use an endoscopic instrument which in this case is schematically shown in a broken line as an endoscope 124 is inserted through the opening 120 in the vicinity of the proximal end 106 of the shaft 102 and slides through the channel 118 towards the opening 122 in the vicinity of the distal end 104 of the shaft 102. The assembly of endoscopic retractor 100 and endoscope 104 as shown in this Figure can then be inserted into the body of a patient. If the operator now wants to move the movable retractor finger 114 and the other movable retractor finger not shown here from the closed position shown here to the open position, he pushes the endoscope 124 in the direction of arrow 126 while holding on to the endoscopic retractor 100. At one point the endoscope 124 will abut against the mounting block 110 and if the endoscope 124 is pushed further forwards the mounting block 110 which is mounted freely movable within the shaft 102 will be pushed towards the distal end 104 of the shaft 102 against the force of the spring 112. Due to the fact that the retractor finger 114 is connected to the mounting block by a connecting stud 117 which is located outside the pivot axis 116 the forward movement of the mounting block 110 is translated into a pivoting motion of the retractor finger 114 in the direction of the arrow 128.

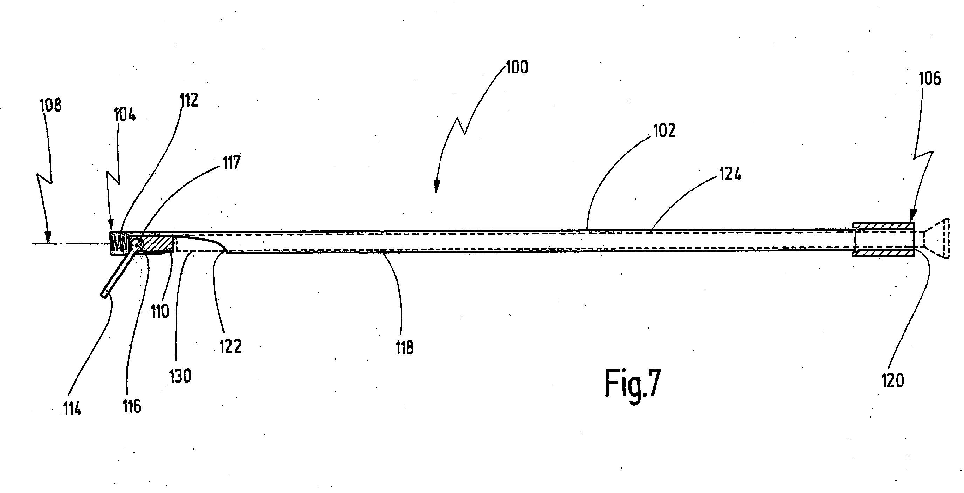

The situation after the movement indicated by the arrows in Fig. 106 is shown in Fig. 7. It can be seen here that a distal end 130 of the endoscope 124 now abuts against the mounting block 110 and has pushed the mounting block relative to the shaft 102 in a distal direction. The spring 112 has been compressed in the process.

The forward motion of the mounting block 110 has been translated into a pivoting motion of the movable retractor finger 114 whereby the same applies to the other retractor finger not shown here. Since the pivot axis 116 of the retractor finger 114 and the pivot axis of the other movable retractor finger that is not visible here are inclined with respect to each other furthermore the two retractor fingers are now at an angle to each other in a plane which in itself is at an angle to the central axis 108 of the shaft 102 and the two retractor fingers are now in a V-shape configuration. Furthermore, the optics at a distal end 130 of the endoscope 124 are now aligned with the opening 122 in the vicinity of the distal end 104 of the shaft 102 so that an operator can view the area surrounding the opening 122 using the endoscope 124.

If an operator now wants to withdraw the assembly of endoscopic retractor 100 and endoscope 124 from the body of a patient he moves the endoscope 124 in a direction opposite to the one indicated in Fig. 6 by arrow 126 relative to the endoscopic retractor 100. If the endoscope 124 has not been locked within the endoscopic retractor 100 this can simply be done by releasing the pressure on the endoscope 124. In this case, the spring 112 will exert the pressure on mounting block 110 and will push it relative to the shaft 102 a proximal direction. This will result in a movement in the opposite direction as indicated in Fig. 6 by arrows 126, 128 and will return the retractor fingers back into the closed position so that the assembly of the endoscopic retractor 100 and the endoscope 124 can be withdrawn from the body of a patient.

## Claims

1. Endoscopic retractor comprising a shaft (12) having a distal end (14), a proximal end (16) and a central axis (22), at least two retractor fingers (24, 26) arranged at the distal end (14) of the shaft (12), whereby the at least two retractor fingers (26) are movable between a closed position in which the at least two movable retractor fingers (26) are substantially parallel to the central axis (22) of the shaft (12) and an open position in which the at least two movable retractor fingers (26) are at an angle to the central axis (22) of the shaft (12), and whereby in the closed position the at least two movable retractor fingers (26) are substantially parallel to each other and in the open position the at least two movable retractor fingers (26) are at an angle to each other in a plane which is at an angle to the central axis (22) of the shaft (12), a handle (18) arranged at the proximal end (16) of the shaft (12), and an actuating element designed as a tube (40) concentric to the shaft (12) connecting the at least two movable retractor fingers (26) with the handle (18), so that the at least two movable retractor fingers (26) can be moved between the open position and the closed position by means of the handle (18) and at least one channel (28) for inserting an endoscopic instrument running along the central axis (22) of the shaft (12) and connecting an opening (30) in the vicinity of the proximal end (16) of the shaft (12) with an opening (32) in the vicinity of the distal end (14) of the shaft (12), wherein the opening (32) in the vicinity of the distal end (14) of the shaft (12) is realized as a cutaway in a sidewall of the shaft (12) and wherein the at least two retractor fingers are mounted distally of the opening (32) in the vicinity of the distal end (14) of the shaft (12) at the distal end (14) of the shaft (12).

2. Endoscopic retractor according to claim 1, **characterized in that** it comprises at least one fixed retractor finger (24) extending from the distal end (14) of the shaft (12) substantially parallel to the central axis (22) of the shaft (12).

3. Endoscopic retractor according to claim 1 or 2, **characterized in that** it comprises sealing means arranged around the inner circumference of the channel (28).

4. Endoscopic retractor according to claim 1 or 3, characterized that it comprises locking means for locking an endoscopic instrument that has been inserted into the channel (28) into place.

5. Endoscopic retractor according to claim 4, **characterized in that** the locking means can lock an endoscopic instrument that has been inserted into the channel (28) into place in any angular orientation with regards to the shaft (12).

6. Endoscopic retractor according to any one of claims 1 to 5, **characterized in that** it comprises image transfer means for transferring images from the distal end (14) of the shaft (12) to the proximal end (16) of the shaft (12).

7. Endoscopic retractor according to any one of claims 1 to 6, **characterized in that** it comprises lighting means for providing light at the distal end (14) of the shaft (12).

8. Endoscopic retractor according to any of claims 1 to 7, **characterized in that** the handle (18) is designed as a lever (20).

9. Endoscope comprising a shaft (62) having a distal end (64), a proximal end (66) and a central axis (72), image transfer means for transferring images from the distal end (64) of the shaft (62) to the proximal end (66) of the shaft (62), a handle (68) arranged at the proximal end (66) of the shaft (62), at least two retractor fingers (74, 76, 90) arranged at the distal end (64) of the shaft (62), whereby the at least two retractor fingers (76, 90) are movable between a closed position in which the at least two movable retractor fingers (76, 90) are substantially parallel to the central axis (72) of the shaft (62) and an open position in which the at least two movable retractor fingers (76, 90) are at an angle to the central axis (72) of the shaft (62), and whereby in the closed position the at least two movable retractor fingers (76, 90) are substantially parallel to each other and in the open position the at least two movable retractor fingers (76, 90) are at an angle to each other in a plane (92) which is at an angle to the central axis (72) of the shaft (62) and an actuating element (69) designed as a tube concentric to the shaft (62) connecting the at least two movable retractor flngers (76, 90) with the handle (68), so that the at least two movable retractor fingers (76, 90) can be moved between the open position and the closed position by means of the handle (68), whereby the distal end of the image transfer means ends in an opening in the shaft (62) in the vicinity of the distal end (64) the shaft (62) through which image information is acquired and which is realized as a cutaway (80) in a sidewall of a shaft and whereby the at least two retractor fingers (76, 90) are mounted distally of the opening in the vicinity of the distal end (64) of the shaft (62) at the distal end (64) of the shaft (62).

10. Endoscope according to claim 9, **characterized in that** it comprises at least one fixed retractor finger (74) extending from the distal end (64) of the shaft (62) substantially parallel to the central axis (72) of the shaft (62).

11. Endoscope according to claim 9 or 10, **characterized in that** the handle (68) is designed as a lever (70).

12. Endoscope according to any one of claims 9 to 11, **characterized in that** it comprises lighting means for providing light at the distal end (64) of the shaft (62).

## Patentansprüche

1. Endoskopischer Retraktor, mit einem Schaft (12), der ein distales Ende (14), ein proximales Ende (18) und eine mittige Achse (22) aufweist, mit zumindest zwei Retraktorfingern (24, 26), die am distalen Ende (14) des Schaftes (12) angeordnet sind, wobei die zumindest zwei Retraktorfinger (26) zwischen einer geschlossenen Position, in der sich die zumindest zwei bewegbaren Retraktorfinger (26) im Wesentlichen parallel zu der mittigen Achse (22) des Schaftes (12) erstrecken und einer offenen Position bewegbar sind, in der die zumindest zwei Retraktorfinger (26) unter einem Winkel zu der mittigen Achse (22) des Schaftes (12) stehen, und wobei die zumindest zwei bewegbaren Retraktorfinger (26) in der geschlossenen Position im Wesentlichen parallel zueinander verlaufen und in der offenen Position die zumindest zwei bewegbaren Retraktorfinger (26) unter einem Winkel zueinander stehen und zwar in einer Ebene, die unter einem Winkel zu der mittigen Achse (22) des Schaftes (12) steht, mit einem Griff (18), der am proximalen Ende (16) des Schaftes (12) angeordnet ist und wobei ein Betätigungselement, das als ein konzentrisch zum Schaft (12) verlaufendes Rohr (40) ausgebildet ist, die zumindest zwei bewegbaren Retraktorfinger (26) mit dem Griff (18) verbindet, so dass die zumindest zwei bewegbaren Retraktorfinger (26) zwischen der offenen Position und der geschlossenen Position mittels des Griffes (18) bewegbar sind, und mit zumindest einem Kanal (28) zum Einbringen eines endoskopischen Instrumentes, der längs der mittigen Achse (22) des Schaftes (12) verläuft und der eine Öffnung (30) in der Nähe des proximalen Endes (16) des Schaftes (12) mit einer Öffnung (32) in der Nähe des distalen Endes (14) des Schaftes verbinder, wobei die Öffnung (32) in der Nähe des distalen Endes (14) des Schaftes (12) als eine Ausnehmung in der Seitenwand des Schaftes (12) ausgebildet ist, und wobei die zumindest beiden Retraktorfinger distalseitig der Öffnung (32) in der Nähe des distalen Endes (14) des Schaftes (12) am distalen Ende (14) des Schaftes (12) ange-bracht sind.

2. Endoskopischer Retraktor nach Anspruch 1, **dadurch gekennzeichnet, dass** er zumindest einen festen Retraktorfinger (24) aufweist, der sich vom distalen Ende (14) des Schaftes (12) im Wesentlichen parallel zu der mittigen Achse (22) des Schaftes (12) erstreckt.

3. Endoskopischer Retraktor nach Anspruch 1 oder 2, **dadurch gekennzeichnet, dass** Dichtungsmittel um den inneren Umgang des Kanales (28) angeordnet sind.

4. Endoskopischer Retraktor nach Anspruch 1 oder 3, **dadurch gekennzeichnet, dass** er Verriegelungsmittel zum Verriegeln eines endoskopischen Instrumentes an Ort und Stelle aufweist, das in den Kanal (28) eingeschoben ist.

5. Endoskopischer Retraktor nach Anspruch 4, **dadurch gekennzeichnet, dass** die Verriegelungsmittel das Endoskop, das in den Kanal (28) eingeschoben ist, in jeglicher Winkelausrichtung bezüglich des Schaftes (12) an Ort und Stelle verriegeln können.

6. Endoskopischer Retraktor nach einem der Ansprüche 1 bis 5, **dadurch gekennzeichnet, dass** er Bildübertragungsmittel aufweist, um Bilder von dem distalen Ende des Schaftes (12) zum proximalen Ende (16) des Schaftes (12) zu übertragen.

7. Endoskopischer Retraktor nach einem der Ansprüche 1 bis 6, **dadurch gekennzeichnet, dass** er Beleuchtungsmittel aufweist, um Licht am distalen Ende (14) des Schaftes (12) bereitzustellen.

8. Endoskopischer Retraktor nach einem der Ansprüche 1 bis 7, **dadurch gekennzeichnet, dass** der Griff (18) als ein Hebel (20) ausgebildet ist.

9. Endoskop, mit einem Schaft (62), der ein distales Ende (64), ein proximales Ende (66) und eine mittige Achse (72) aufweist, mit Bildübertragungsmitteln zum Übertragen von Bildern von dem distalen Ende (64) des Schaftes (22) zum proximalen Ende (66) des Schaftes, mit einem Griff (68), der am proximalen Ende (66) des Schaftes (62) angeordnet ist, mit zumindest zwei Retraktorfingern (74, 76, 90), die am distalen Ende (64) des Schaftes (62) angeordnet sind, wobei die zumindest zwei Retraktorfinger (76, 90) zwischen einer geschlossenen Position, in der die zumindest zwei bewegbaren Retraktorfinger (76, 90) sich im Wesentlichen parallel zu der mittigen Achse (72) des Schaftes (62) erstrecken und einer offenen Position bewegbar sind, in der die zumindest zwei bewegbaren Retraktorfinger (76, 90) unter einem Winkel zu der mittigen Achse (72) des Schaftes (62) stehen, und wobei in der geschlossenen Position die zumindest zwei bewegbaren Retraktorfinger (76, 90) im Wesentlichen parallel zueinander verlaufen und in der offenen Position die zumindest zwei bewegbaren Retraktorfinger (76, 90) unter einem Winkel zueinander stehen und zwar in einer Ebene (92), die unter einem Winkel zu der mittigen Achse (72) des Schaftes (62) steht, und wobei ein Betätigungselement (69), das als ein konzentrisch zum Schaft (62) verlaufendes Rohr ausgebildet ist, die zumindest zwei bewegbaren Retraktorfinger (76, 90) mit dem Griff (68) verbindet, so dass die zumindest zwei bewegbaren Retraktorfinger (76, 90) mittels des Griffes (68) zwischen der offenen Position und der geschlossenen Position bewegbar sind, wobei das distale Ende der Bildübertragungsmittel in einer Öffnung im Schaft (62) in der Nähe des distalen Endes (64) des Schaftes (62) endet, durch die die Bildinformation erhalten wird, und die als Ausnehmung (80) in einer Seitenwand des Schaftes ausgebildet ist, und wobei die zumindest zwei Reaktrorfinger (72, 90) distalseitig der Öffnung in der Nähe des distalen Endes (64) des Schaftes (62) am distalen Ende (64) des Schaftes (62) befestigt sind.

10. Endoskop nach Anspruch 9, **dadurch gekennzeichnet, dass** es zumindest einen festen Retraktorfinger (74) aufweist, der sich vom distalen Ende (64) des Schaftes (62) im Wesentlichen parallel zu der mittigen Achse (72) des Schaftes (62) erstreckt.

11. Endoskop nach Anspruch 9 oder 10, **dadurch gekennzeichnet, dass** der Griff (68) als ein Hebel (70) ausgebildet ist.

12. Endoskop nach einem der Ansprüche 9 bis 11, **dadurch gekennzeichnet, dass** Leuchtmittel vorhanden sind, die Licht am distalen Ende (64) des Schaftes (62) bereitstellen.

## Revendications

1. Ecarteur endoscopique comprenant un arbre (12) présentant une extrémité distale (14), une extrémité proximale (16) et un axe central (22), au moins deux doigts d'écarteur (24, 26) agencés au niveau de l'extrémité distale (14) de l'arbre (12), grâce à quoi les au moins deux doigts d'écarteur (26) sont mobiles entre une position fermée dans laquelle les au moins deux doigts d'écarteur mobiles (26) sont sensiblement parallèles à l'axe central (22) de l'arbre (12) et une position ouverte dans laquelle les au moins deux doigts d'écarteur mobiles (26) forment un certain angle par rapport à l'axe central (22) de l'arbre (12), et grâce à quoi dans la position fermée les au moins deux doigts d'écarteur mobiles (26) sont sensiblement parallèles l'un à l'autre et dans la position ouverte les au moins deux doigts d'écarteur mobiles (26) forment un certain angle l'un par rapport à l'autre dans un plan qui forme un certain angle par rapport à l'axe central (22) de l'arbre (12), une poignée (18) agencée au niveau de l'extrémité proximale (16) de l'arbre (12), et un élément d'actionnement conçu sous la forme d'un tube (40) concentrique par rapport à l'arbre (12) reliant les au moins deux doigts d'écarteur mobiles (26) à la poignée (18), de sorte que les au moins deux doigts d'écarteur mobiles (26) peuvent être déplacés entre la position ouverte et la position fermée au moyen de la poignée (18) et au moins un canal (28) destiné à insérer un instrument endoscopique courant le long de l'axe central (22) de l'arbre (12) et reliant une ouverture (30) à proximité de l'extrémité proximale (16) de l'arbre (12) à une ouverture (32) à proximité de l'extrémité distale (14) de l'arbre (12), où l'ouverture (32) à proximité de l'extrémité distale (14) de l'arbre (12) est ménagée sous la forme d'un dégagement dans une paroi latérale de l'arbre (12) et où les au moins deux doigts d'écarteur sont installés de manière distale par rapport à l'ouverture (32) à proximité de l'extrémité distale (14) de l'arbre (12) au niveau de l'extrémité distale (14) de l'arbre (12).

2. Ecarteur endoscopique selon la revendication 1, **caractérisé en ce qu'**il comprend au moins un doigt d'écarteur fixe (24) s'étendant depuis l'extrémité distale (14) de l'arbre (12) de manière sensiblement parallèle à l'axe central (22) de l'arbre (12).

3. Ecarteur endoscopique selon la revendication 1 ou 2, **caractérisé en ce qu'**il comprend un moyen d'étanchéité agencé autour de la circonférence interne du canal (28).

4. Ecarteur endoscopique selon la revendication 1 ou 3, **caractérisé en ce qu'**il comprend un moyen de blocage destiné à bloquer en position un instrument endoscopique qui a été inséré dans le canal (28).

5. Ecarteur endoscopique selon la revendication 4, **caractérisé en ce que** le moyen de blocage peut bloquer en position un instrument endoscopique qui a été inséré dans le canal (28) selon une orientation angulaire quelconque par rapport à l'arbre (12).

6. Ecarteur endoscopique selon l'une quelconque des revendications 1 à 5, **caractérisé en ce qu'**il comprend un moyen de transfert d'images destiné transférer des images depuis l'extrémité distale (14) de l'arbre (12) jusqu'à l'extrémité proximale (16) de l'arbre (12).

7. Ecarteur endoscopique selon l'une quelconque des revendications 1 à 6, **caractérisé en ce qu'**il comprend un moyen d'éclairage destiné à fournir de la lumière au niveau de l'extrémité distale (14) de l'arbre (12).

8. Ecarteur endoscopique selon l'une quelconque des revendications 1 à 7, **caractérisé en ce que** la poignée (18) est conçue sous la forme d'un levier (20).

9. Endoscope comprenant un arbre (62) présentant une extrémité distale (64), une extrémité proximale (66) et un axe central (72), un moyen de transfert d'images destiné à transférer des images depuis l'extrémité distale (64) de l'arbre (62) jusqu'à l'extrémité proximale (66) de l'arbre (62), une poignée (68) agencée au niveau de l'extrémité proximale (66) de l'arbre (62), au moins deux doigts d'écarteur (74, 76, 90) agencés au niveau de l'extrémité distale (64) de l'arbre (62), grâce à quoi les au moins deux doigts d'écarteur (76, 90) sont mobiles entre une position fermée dans laquelle les au moins deux doigts d'écarteur mobiles (76, 90) sont sensiblement parallèles à l'axe central (72) de l'arbre (62) et une position ouverte dans laquelle les au moins deux doigts d'écarteur mobiles (76, 90) forment un certain angle par rapport à l'axe central (72) de l'arbre (62), et grâce à quoi dans la position fermée les au moins deux doigts d'écarteur mobiles (76, 90) sont sensiblement parallèles l'un à l'autre et dans la position ouverte les au moins deux doigts d'écarteur mobiles (76, 90) forment un certain angle l'un par rapport à l'autre dans un plan (92) qui forme un certain angle par rapport à l'axe central (72) de l'arbre (62) et un élément d'actionnement (69) conçu sous la forme d'un tube concentrique par rapport à l'arbre (62) reliant les au moins deux doigts d'écarteur mobiles (76, 90) à la poignée (68), de sorte que les au moins deux doigts d'écarteur mobiles (76, 90) peuvent être déplacés entre la position ouverte et la position fermée au moyen de la poignée (68), grâce à quoi l'extrémité distale du moyen de transfert d'images se termine dans une ouverture dans l'arbre (62) à proximité de l'extrémité distale (64) de l'arbre (62) au travers laquelle des informations d'images sont acquises et qui est ménagée sous la forme d'un dégagement (80) dans une paroi latérale d'un arbre et grâce à quoi les au moins deux doigts d'écarteur (76, 90) sont installés de manière distale par rapport à l'ouverture à proximité de l'extrémité distale (64) de l'arbre (62) au niveau de l'extrémité distale (64) de l'arbre (62).

10. Endoscope selon la revendication 9, **caractérisé en ce qu'**il comprend au moins un doigt d'écarteur fixe (74) s'étendant depuis l'extrémité distale (64) de l'arbre (62) de manière sensiblement parallèle à l'axe central (72) de l'arbre (62).

11. Endoscope selon la revendication 9 ou 10, **caractérisé en ce que** la poignée (68) est conçue sous la forme d'un levier (70).

12. Endoscope selon l'une quelconque des revendications 9 à 11, **caractérisé en ce qu'**il comprend un moyen d'éclairage destiné à fournir de la lumière au niveau de l'extrémité distale (64) de l'arbre (62).
